(19) Europäisches Patentamt
European Patent Office
Office européen des brevets

(11) Veröffentlichungsnummer : **0 046 840**
**B1**

(12) # EUROPÄISCHE PATENTSCHRIFT

(45) Veröffentlichungstag der Patentschrift : **05.10.83**

(51) Int. Cl.³ : **C 07 C 57/05**, C 07 C 51/377, B 01 J 27/18

(21) Anmeldenummer : **81102670.7**

(22) Anmeldetag : **09.04.81**

(54) **Verfahren zur oxydativen Dehydrierung von Isobuttersäure zu Methacrylsäure.**

(30) Priorität : **28.08.80 DE 3032423**
**02.02.81 DE 3103410**

(43) Veröffentlichungstag der Anmeldung : **10.03.82 Patentblatt 82/10**

(45) Bekanntmachung des Hinweises auf die Patenterteilung : **05.10.83 Patentblatt 83/40**

(84) Benannte Vertragsstaaten : **BE DE FR GB IT NL**

(56) Entgegenhaltungen :
**EP A 0 005 769**
**EP A 0 013 578**
**DE A 2 739 779**
**Patents Abstracts of Japan Band 4, Nr. 151, 23. Oktober 1980 Seite 110C28**
**Chemical Abstracts Band 95, Nr. 2, 1981 Columbus, Ohio, USA MITSUBISHI CHEMICAL IND. « Manufacture of methacrylic acid» Seite 19, Spalte 1, Abstract Nr. 8008r**
**Chemical Abstracts Band 89, Nr. 12, 1978 Columbus, Ohio, USA T. ONODA et al. « Mathacrylic acid» Seite 12, Spalte 1, Abstract Nr. 90435k**
**Chemical Abstracts Band 88, Nr. 20, 1978 Columbus, Ohio, USA T. ONODA et al. « Heteropolyacid » Seite 13, Spalte 1, Abstract Nr. 137168x**
**Chemical Abstracts Band 83, Nr. 6, 1975 Columbus, Ohio, USA T. OTAKI et al. « Methacrylic acid from Isobutyric acid» Seite 16, Spalte 2, Abstract Nr. 44008b**

(73) Patentinhaber : **Röhm GmbH**
**Kirschenallee Postfach 4242**
**D-6100 Darmstadt 1 (DE)**

(72) Erfinder : **Gruber, Wilhelm, Dr.**
**Peter-Behrens-Strasse 34**
**D-6100 Darmstadt (DE)**
Erfinder : **Schröder, Günter, Dr.**
**Leipziger Strasse 7**
**D-6105 Ober-Ramstadt (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**EP 0 046 840 B1**

Jouve, 18, rue St-Denis, 75001 Paris, France

Verfahren zur oxydativen Dehydrierung von Isobuttersäure zu Methacrylsäure

Die Erfindung betrifft ein Verfahren zur oxydativen Dehydrierung von Isobuttersäure zu Methacrylsäure mittels Kontaktkatalyse.

Der Synthese der Methacrylsäure und ihrer Ester zur Herstellung von Polymerisaten kommt große wirtschaftliche Bedeutung zu. Neben dem überwiegend durchgeführten großtechnischen Verfahren, das ausgehend von Aceton durch Verseifung von Acetoncyanhydrin zum wichtigsten Monomeren in der Methacrylatserie, dem Methylmethacrylat führt, haben in jüngerer Zeit auch Synthesewege, an deren Anfang Isobutyraldehyd oder Olefine, wie Isobutylen, Aethylen oder Propylen stehen, das Interesse der Technik gefunden.

Ausgehend von dem z. B. aus Isobutylen oder Isobutyraldehyd zugänglichen Methacrolein wird in der DE-OS 25 17 148 die Herstellung von Methacrylsäure durch katalytische Gasphasenoxydation mittels Sauerstoff beschrieben.

Der in dieser Reaktion verwendete Katalysator entspricht der Formel

$$Mo_{12}P_aX_bY_cW_dO_e,$$

wobei X Vanadin, Niob und/oder Tantal und Y Caesium, Kalium und/oder Thallium bedeuten und a und b Werte von 0,1 bis 10, c Werte von 0,1 bis 8 und d Werte von 0 bis 10 haben und der Wert von e von der Wertigkeit der übrigen Atome abhängt und die Summe von a + b + c einen Wert von 0,3 bis 20 besitzt.

Zur oxydativen Dehydrierung von Isobuttersäure zu Methacrylsäure ist eine Reihe von Katalysatoren vorgeschlagen worden. In der Jap. Offenlegungsschrift 7 504 017 wird ein Katalysator, der Molybdän, Phosphor, Thallium und Sauerstoff enthält, für die Gasphasendehydrierung der Isobuttersäure vorgeschlagen.

Die Verwendung von Molybdo- oder Molybdovanadophosphorsäure, die mit Nickel-, Cobalt- oder Alkalimetallsulfaten behandelt wurden, als Katalysatoren zur Oxydehydrierung von Isobuttersäure in der Gasphase, ist Gegenstand der Jap. Offenlegungsschrift 7 504 014.

Vorgeschlagen wurde auch die oxydative Dehydrierung von Alkancarbonsäuren und -Estern zu α,β-ungesättigten aliphatischen Säuren und Estern mit calziniertem Eisenphosphat/Bleiphosphat als Dehydrierungskatalysator (DE-OS 24 50 878).

Die Dehydrierung dieser Verbindungsklasse mit Sauerstoff unter Verwendung von calciniertem gefälltem Phosphat, das Wismut, Eisen und ggf. Blei enthält, wird in der DE-OS 21 18 904 gelehrt.

Die Verfahren des Standes der Technik konnten indessen nicht völlig befriedigen. Unter modernen Gesichtspunkten kommt neben den reinen Anlagekosten der Bilanz aus Energie- und Rohstoffaufwand im Verhältnis zur Raumzeitausbeute und zur Selektivität der Verfahren eine entscheidende Bedeutung zu.

So bringt z. B. die Umsetzung an den Kontakten auf Eisen-Phosphat-Basis relativ niedrige Isobuttersäureumsätze, d. h. nicht umgesetzte Isobuttersäure müßte in das Verfahren zurückgeführt werden. Wegen der sehr ähnlichen physikalischen Eigenschaften von Isobutter- und Methacrylsäure ist eine Trennung dieser beiden Verbindungen in technischem Maßstab problematisch und in jedem Falle teuer.

In der DE-OS 25 50 979 wird die Verwendung von Feststoff-Katalysatoren, die im wesentlichen aus Molybdän und/oder Wolfram bzw. ihren Oxiden bestehen, bei Temperaturen zwischen 300 und 500 °C beschrieben.

Aus der DE-OS 27 22 375 ist ein hydrothermisches Verfahren zur Herstellung von Katalysatoren auf der Basis von Heteropolysäuren aus einem Oxid bzw. einer Oxysäure des Molybdäns, Vanadiums, Phosphors und ggf. des Wolframs bekannt, das sich zur oxydativen Dehydrierung von Isobuttersäure bzw. seines Methylesters und von Methacrolein eignet.

Untersuchungen mit Heteropolysäure-Kontakten zeigten jedoch, daß relativ hohe Umsätze mit guter Selektivität dann zu erreichen sind, wenn oberhalb 330 °C und in einem relativ engen Temperaturbereich (bei etwa 330 bis 350 °C) gearbeitet wird. Unterhalb dieses Temperaturbereichs gehen die Umsätze stark zurück, während oberhalb 350 °C der Kontakt offenbar irreversibel geschädigt wird, so daß sowohl Umsatz als auch Selektivität der Reaktion stark zurückgehen. Die Einhaltung derart enger Temperaturbereiche auf relativ hohem Temperaturniveau unter den Bedingungen der heterogenen Katalyse ist bei vernünftigem Aufwand technisch nur schwer zu realisieren, d. h. in der Praxis besitzen derartige Katalysatorsysteme unter den genannten Reaktionsbedingungen nur eine geringe Lebensdauer.

Es wurde nun gefunden, daß sich das Verfahren zur oxydativen Dehydrierung von Isobuttersäure zu Methacrylsäure mit ausgezeichneter Ausbeute und hoher Selektivität durchführen läßt, wenn sulfatfreie Metalloxid-Katalysatoren auf der Basis von Molybdänoxid verwendet werden, die Außerdem Vanadin, Phosphor und bestimmte, im Anspruch 1 aufgezählte Metallkationen enthalten. Die erfindungsgemäß als Katalysatoren geeigneten Verbindungen lassen sich durch die Formel

$$M_aMo_bV_cP_dO_e$$

wiedergeben, worin M, a, b, c, d und e die im Anspruch 1 angegebene Bedeutung haben. Eine bevorzugte

Gruppe von Metallen M umfaßt Magnesium, Calcium und Aluminium.

Besonders bevorzugt ist die Gruppe von Katalysatoren, worin b den Wert 12, d ungefähr den Wert von a und c einen Wert von ca. 0,75 a besitzt. Genannt sei beispielsweise ein System der angenäherten Zusammensetzung $A_{1,5-2}Mo_{12}V_{1,5}P_2O_{45-46}$, wobei A für ein Alkalimetall der Gruppe Kalium, Rubidium und Caesium steht, insbesondere der Zusammensetzung

$$Cs_{1,5-2}Mo_{12}V_{1,5}P_2O_{45,8}.$$

Zur Herstellung eines Katalysators kann von einer Molybdänverbindung, wie z. B. dem Molybdänoxid bzw. der Molybdänsäure $H_2MoO_4$ oder einem Salz, wie z. B. dem Ammoniumsalz, einer Verbindung des 5-wertigen Phosphors, wie z. B. Phosphorsäure oder Polyphosphorsäure bzw. ihren Salzen, oder dem Phosphorpentoxid, einem Salz des Vanadins, wie z. B. einem Nitrat, Carbonat, Sulfat oder Halogenid oder dem Salz einer organischen Säure oder dem Oxid des Vanadins und einem Salz oder Hydroxid der im Anspruch gekennzeichneten Metalle M, beispielsweise einem Metallhalogenid, -carbonat, -nitrat, -sulfat oder dem Salz einer organischen Säure ausgegangen werden. Sofern eines der erwähnten Metalle in Form seines Sulfates zu dem Bildungsgemisch für den Katalysator gegeben wird, muß dafür Sorge getragen werden, daß der Sulfatanteil während der Herstellung des Katalysators im wesentlichen vollständig abgetrennt wird. Vorzugsweise wird keine der Bildungskomponenten in Form des Sulfats eingesetzt.

Die Herstellung des Katalysators kann sich an die bekannten Verfahren zur Herstellung von Metalloxid-Katalysatoren komplexer Zusammensetzung anschließen. Beispielsweise erhält man das Katalysatorsystem durch Mischen der in einem flüssigen Reaktionsmedium, wie z. B. in Wasser, gelösten bzw. suspendierten Komponenten, wobei auf möglichst homogene Verteilung zu achten ist. Vorteilhafterweise kann insbesondere bei Verwendung von Ammoniumverbindungen der wäßrigen Lösung noch ein Alkanolamin, beispielsweise Monoäthanolamin, zugefügt werden. Das flüssige Reaktionsmedium kann anschließend durch Verdampfen, vorzugsweise unter Erhitzen, entfernt werden. Bei der Verwendung von Wasser als Reaktionsmedium kann die Entfernung des Wassers bei 100 bis 150 °C, speziell bei ca. 120 °C, vorgenommen werden. Anschließend findet vorteilhafterweise eine Calcinierung des Katalysatorsystems, beispielsweise bei Temperaturen zwischen 200 und 400 °C statt, die sich über ca. 1 bis 2 Stunden erstrecken kann.

Besonders bevorzugt ist die Ausführungsform unter Verwendung eines festen Trägers für das erfindungsgemäße Katalysatorsystem. Bevorzugt sind Träger auf der Basis von Sauerstoffverbindungen des Aluminiums, Titans und insbesondere des Siliciums, bzw. Mischungen verschiedener derartiger Sauerstoffverbindungen. Besonders bevorzugt ist ein Trägersystem auf der Basis von Siliciumdioxid und « aktiviertem Siliciumdioxid » (vgl. Unger, Angew. Chemie 84, 331 [1972]), insbesondere auf der Basis von Kieselgur und Aerosil®. Das bevorzugte Mischungsverhältnis von Siliciumdioxid (z. B. Kielselgur) und aktiviertem Siliciumdioxid (z. B. Aerosil®) beträgt 14 : 2 bis 9 : 2, speziell 5 : 1. Das Verhältnis Katalysator zu Träger kann dabei in gewissen Grenzen schwanken. Im allgemeinen macht der Katalysator 5 bis 80 Gew.-%, vorzugsweise 20 bis 70 Gew.-%, bezogen auf das Gesamtgewicht (Katalysator plus Träger), aus.

Das Produkt kann in geeigneter Weise für die Verwendung als Katalysatorsystem präpariert werden, beispielsweise durch Granulieren, Körnen, Tablettieren usw. An die Körnung bzw. Granulation und die Tablettierung kann sich erneut ein Temperungsschritt, beispielsweise zwischen 300 und 500 °C anschließen, der sich beispielsweise über ca. 1 bis ca. 24 h erstrecken kann.

Das katalytisch wirksame System kann per se verwendet werden oder vorzugsweise in Abmischung mit einem inerten Material, wie z. B. Quarz oder Zirkondioxid. Diese letztere Ausführungsart hat ökonomische und technologische Vorteile ; beispielsweise kann eine Verbesserung des Temperaturprofils, Stabilisierung der Reaktorzustände usw. erreicht werden.

Das Katalysatorsystem gemäß der vorliegenden Erfindung läßt sich bei der oxydativen Dehydrierung von Isobuttersäure innerhalb eines relativ weiten Temperaturbereiches zur Anwendung bringen, ohne daß eine reduzierte Wirksamkeit bzw. eine Inaktivierung beobachtet würde.

Vorzugsweise wird das Verfahren zwischen 300 und 450 °C, speziell zwischen 340 und 370 °C durchgeführt. Die Verweilzeiten liegen dabei im allgemeinen zwischen 0,1 und 5 sec. Wenn also im Zuge des katalytischen Verfahrens Temperaturen auftreten, die oberhalb des optimalen Temperaturbereiches liegen, so führt dies in der Regel nicht zur irreversiblen Inaktivierung des Katalysatorsystems. Die erfindungsgemäßen Katalysatorsysteme zeichnen sich durch eine hervorragende Raum-Zeitausbeute bei hoher Selektivität aus. Das oxydative Dehydrierungsverfahren gemäß der vorliegenden Erfindung bedient sich des Sauerstoffs als Oxydationsmittel, beispielsweise in Form von Luft. Die Reaktion wird zweckmäßig als Gasphasenreaktion durchgeführt, wobei mit Isobuttersäure und Sauerstoff in der Gasphase, vorzugsweise mit einem Gasgemisch aus Isobuttersäure, Wasser, Sauerstoff und Stickstoff gearbeitet wird.

Das Verhältnis der Komponenten in der angegebenen Reihenfolge bewegt sich vorteilhaft innerhalb der folgenden Grenzen : (1 – 2,5) : (0 – 3) : (1 – 2) : (4 – 10) Mol, besonders bevorzugt im Verhältnis 1 : 2 : 1,7 : 20 Mol. Die Anwensenheit von Wasser wirkt sich erfahrungsgemäß günstif auf das Reaktionsgeschehen aus. Das erfindungsgemäße Verfahren kann in üblichen Reaktoren, beispielsweise unter

Anwendung von Druck, durchgeführt werden. Die infrage kommenden Drücke liegen bei 0,5 bis 5 bar, vorzugsweise bei 0,8 bis 2,0 bar. Eine Erhöhung des Sauerstoffanteiles während der Reaktion kann zu einem nicht unerheblichen Temperaturanstieg führen, der aber wegen der unerwartet hohen Stabilität des Katalysatorsystems in der Regel nicht zu dessen Inaktivierung führt. Erfindungsgemäß können die Katalysatoren im Festbett oder im Fließbett eingesetzt werden. Die folgenden Beispiele dienen zur Erläuterung der Erfindung. Prozentangaben — soweit nicht anders definiert — beziehen sich auf das Gewicht.

Die angegebenen Umsätze bzw. Selektivitäten sind definiert:

$$\text{Umsatz Isobuttersäure (\%)} = \frac{\text{Mol umgesetzte Isobuttersäure}}{\text{Mol eingesetzte Isobuttersäure}} \cdot 100$$

$$\text{Selektivität der Methacrylsäurebildung (\%)} = \frac{\text{Mol gebildete Methacrylsäure}}{\text{Mol umgesetzte Isobuttersäure}} \cdot 100$$

A. Herstellung der Katalysatoren

1. Zu einer Lösung von 106 g $(NH_4)_6Mo_7O_{24} \cdot 4H_2O$ in 300 ml einer 10 %-igen Ammoniaklösung werden 11,52 g 85 %-ige Orthophosphorsäure, gelöst in 100 ml Wasser, gegeben und das Ganze in 19,4 g Caesiumnitrat, die in 200 ml Wasser gelöst sind, eingerührt. Hierzu kommen noch 8,8 g $NH_4VO_3$, gelöst in 140 ml 10 %-igem wäßrigem Monoäthanolamin. Die so erhaltene Lösung wird nach Zugabe von 40,84 g gereinigtem und geglühtem Kieselgur und 8,18 g Aerosil® 200 als Kontaktträgern unter Rühren eingedampft. Bei 120 °C wird die feste Katalysatormasse 7 Stunden lang getrocknet und anschließend 3 Stunden lang bei 300 °C calciniert. Der so erhaltene 70 %ige $Cs_2Mo_{12}V_{1,5}P_2O_{45,8}$-Kontakt auf Kieselgur/Aerosil® 200 stellt eine sehr harte Masse dar, aus der für die Reaktorfüllung Kontaktkörner in einer Korngröße von ca. 3 mm hergestellt werden.

2. Für die Herstellung des Kontaktes $Cs_{1,5}Mo_{12}V_{1,5}P_2O_{45,5}$ wird wie nach 1. verfahren, wobei die eingesetzte Caesiumnitratmenge auf 14,62 g reduziert ist.

3. Zur Herstellung der in der Tabelle aufgeführten und geprüften Katalysatoren wird wie nach 1. verfahren, wobei anstelle des Cs-Salzes die äquivalente Menge des in der Tabelle genannten Metalles in Form eines Salzes zugegeben wird.

B. Verfahren zur Oxydehydrierung von Isobuttersäure

Beispiel 1

Über 14,9 ml eines gemäß A) 1 hergestellten Kontaktes in einem Stahlreaktor wurde bei 350 °C und einer Verweilzeit von 0,9 sec ein Gasgemisch von Isobuttersäure, Wasser, Sauerstoff und Stickstoff im Molverhältnis 1 : 2 : 1, 8 : 20 geleitet. Nach einer Aktivierungszeit von 20 Stunden wurden Methacrylsäureausbeuten von 69 % d. Th. bei Isobuttersäureumsätzen von 100 % erreicht.

Beispiel 2

In einem Glasreaktor mit 20 ml des nach A) 1 hergestellten Kontaktes wurde eine Oxydehydrierung von Isobuttersäure bei 360 °C, einer Verweilzeit von 0,6 sec bei Einsatz eines Gasgemisches von Isobuttersäure, Wasser, Sauerstoff und Stickstoff im Molverhältnis 1 : 2 : 1, 8 : 20 durchgeführt. Dabei erreichten über einen Zeitraum von 150 Stunden die Isobuttersäureumsätze 99,5 % und die Methacrylsäureselektivitäten 70 %.

Beispiel 3

Ein dampfförmiges Gemisch mit Isobuttersäure Methacrylsäure, Wasser und Sauerstoff (als Luft) im Molverhältnis 0,3 : 0,49 : 0,98 : 1,1 wurde über den nach A) 1 hergestellten Katalysator, in einem Glasreaktor als zweite Stufe in einer 2-Stufenumsetzung bei 350 °C und einer Verweilzeit von 0,4 sec geleitet. Nach dem Durchgang wurde für beide Stufen ein Gesamtisobuttersäureumsatz von > 98 % und eine Methacrylsäureselektivität von 71,3 ermittelt.

Beispiel 4

Mit dem nach A) 2 hergestellten Kontakt wurden bei Prüfungen der Isobuttersäureoxydehydrierung nach Beispiel 1 praktisch die gleichen Isobuttersäureumsätze und Methacrylsäureausbeuten wie mit dem Kontakt nach A) 1 erhalten.

Beispiele 5 bis 17

In der anliegenden Tabelle sind Ergebnisse zusammengestellt, die mit weiteren Kontakten der

allgemeinen Formel $M_aMo_bV_cP_dO_e$ bei der Oxydehydrierung von Isobuttersäure zu Methacrylsäure erhalten wurden.

Hierzu wurden im Temperaturbereich von 340 bis 370 °C Gemische von Isobuttersäure, Wasser und Sauerstoff (als Luft) im Molverhältnis von 1 : 2 : 1,7 bei Verweilzeiten von 0,3 bis 0,4 sec über die Kontakte geleitet.

Tabelle

| Beispiel Nr. | Katalysatortemperatur °C | Katalysator | Umsatz Isobuttersäure | Selektivität Methacrylsäure |
|---|---|---|---|---|
| 5 | 350 | $Na_2Mo_{12}V_{1,5}P_2O_{45,8}$ | 70 | 60 |
| 6 | 370 | $K_2Mo_{12}V_{1,5}P_2O_{45,8}$ | 95 | 64 |
| 7 | 360 | $Rb_2Mo_{12}V_{1,5}P_2O_{45,8}$ | 98 | 66 |
| 8 | 340 | $BeMo_{12}V_{1,5}P_2O_{45,8}$ | 97,8 | 57 |
| 9 | 340 | $MgMo_{12}V_{1,5}P_2O_{45,8}$ | 86,4 | 63,4 |
| 10 | 370 | $CaMo_{12}V_{1,5}P_2O_{45,8}$ | 98,2 | 65,5 |
| 11 | 340 | $BaMo_{12}V_{1,5}P_2O_{45,8}$ | 80 | 57 |
| 12 | 370 | $Ag_2Mo_{12}V_{1,5}P_2O_{45,8}$ | 96 | 60 |
| 13 | 350 | $ZnMo_{12}V_{1,5}P_2O_{46,8}$ | 96,5 | 57 |
| 14 | 360 | $Al_{0,67}Mo_{12}V_{1,5}P_2O_{45,3}$ | 99,2 | 65,5 |
| 15 | 360 | $PbMo_{12}V_{1,5}P_2O_{45,8}$ | 70 | 60 |
| 16 | 340 | $SnMo_{12}V_{1,5}P_2O_{45,8}$ | 80 | 45 |
| 17 | 350 | $TlMo_{12}V_{1,5}P_2O_{45,8}$ | 77,8 | 64,8 |

Beispiele 18-24

Analog der Arbeitsweise gemäß A3 werden vier Katalysatoren A bis D mit folgender Zusammensetzung hergestellt :

A) $MnMo_{12}V_{1,5}P_2O_{45,8}$
B) $Fe_{0,67}Mo_{12}V_{1,5}P_2O_{45,8}$
C) $CoMo_{12}V_{1,5}P_2O_{45,8}$
D) $NiMo_{12}V_{1,5}P_2O_{45,8}$

Im Temperaturbereich von 320-350 °C wurden über die Katalysatoren A bis D Gemische von Isobuttersäure, Wasser und Luftsauerstoff im Molverhältnis 1 : 2 : 1,7 und einer Verweilzeit von 0,3 sec geleitet. In der nachfolgenden Tabelle sind die erreichten Werte für den Isobuttersäureumsatz und die Selektivität an Methacrylsäure verzeichnet ;

| Beisp. Nr. | Katalysator | Katalysator-temperatur °C | Umsatz an Isobutter-säure (%) | Selektivität an Methacryl-säure (%) |
|---|---|---|---|---|
| 18 | A | 330 | 84,8 | 64,9 |
| 19 | A | 340 | 93,0 | 62,3 |
| 20 | B | 330 | 81,8 | 66,1 |
| 21 | B | 350 | 95,9 | 62,3 |
| 22 | C | 340 | 91,1 | 65,5 |
| 23 | C | 350 | 96,1 | 64,5 |
| 24 | D | 350 | 93,6 | 58,4 |

Beispiel 25

Über 13 ml eines auf Kieselgur niedergeschlagenen Katalysators der Zusammensetzung C gemäß Beispiele 18-24 wurde bei 360 °C mit einer Verweilzeit von 0,5 sec ein Gasgemisch aus Isobuttersäure, Wasser, Sauerstoff und Stickstoff im Molverhältnis 1 : 2 : 1,5 : 20 geleitet. Bei einem Umsatz von 99,8 % der eingesetzten Isobuttersäure wurde Methacrylsäure in einer Selektivität von 74,1 % erzeugt.

**Ansprüche**

1. Verfahren zur oxydativen Dehydrierung von Isobuttersäure zu Methacrylsäure unter Verwendung eines sulfatfreien Katalysators auf der Basis von Molybdänoxid, der zusätzlich Vanadin, Phosphor und ein weiteres Metall enthält, dadurch gekennzeichnet, daß der Katalysator der Formel

$$M_aMo_bV_cP_dO_e$$

entspricht, worin M eines der Elemenke K, Rb, Cs, Be, Mg, Ba, Zn, Ca, Ag, Al, Ti, Pb, Mn, Fe, Co, Ni oder Sn bedeutet und a einen Wert von 0,4 bis 2,5, b den Wert 12, c einen Wert von 1 bis 2 und d einen Wert von 0,5 bis 3 haben und e sich aus den Wertigkeiten und Anteilen der anderen Elemente von selbst ergibt.

2. Verfahren gemäß Anspruch 1, dadurch gekennzeichnet, daß der Metalloxid-Katalysator durch die Formel

$$A_{1,5-2}Mo_{12}V_{1,5}P_2O_{45-46}$$

worin A für ein Alkalimetall der Gruppe Kalium, Rubidium und Caesium steht, wiedergegeben wird.

3. Verfahren gemäß den Ansprüchen 1 und 2, dadurch gekennzeichnet, daß der Metalloxid-Katalysator in Verbindung mit einem festen Träger auf Basis Siliciumdioxid/aktiviertes Siliciumdioxid, vorzugsweise auf Basis Kieselgur/Aerosil®, angewendet wird.

4. Verfahren gemäß Anspruch 3, dadurch gekennzeichnet, daß das Verhältnis Siliciumdioxid/aktiviertes Siliciumdioxid im Träger 14 : 2 bis 9 : 2, vorzugsweise 5 : 1, beträgt.

5. Verfahren gemäß den Ansprüchen 1 bis 4, dadurch gekennzeichnet, daß der Metalloxid-Katalysator 5 bis 80 Gew.-%, vorzugsweise 20 bis 70 Gew.-%, bezogen auf das Gesamtgewicht (Träger plus Metalloxid-Katalysator), ausmacht.

6. Verfahren gemäß den Ansprüchen 1 bis 5, dadurch gekennzeichnet, daß man den Katalysator bei Temperaturen zwischen 200 und 400 °C calciniert.

7. Verfahren gemäß den Ansprüchen 1 bis 6, dadurch gekennzeichnet, daß es als Gasphasenreaktion durchgeführt wird.

8. Verfahren gemäß Anspruch 7, dadurch gekennzeichnet, daß mit einem Gasgemisch aus Isobuttersäure, Wasser, Sauerstoff und Stickstoff gearbeitet wird.

9. Verfahren gemäß den Ansprüchen 7 und 8, dadurch gekennzeichnet, daß das Molverhältnis Isobuttersäure zu Wasser zu Sauerstoff zu Stickstoff (1-2,5) : (0-3) : (1-2) : (4-10), vorzugsweise 1 : 2 : 1, 7 : 20, beträgt.

10. Verfahren gemäß den Ansprüchen 1 bis 9, dadurch gekennzeichnet, daß die Umsetzung bei Temperaturen von 300 bis 450 °C, vorzugsweise bei 340 bis 370 °C, durchgeführt wird.

11. Verfahren gemäß Anspruch 8, dadurch gekennzeichnet, daß man das Gasgemisch bei einer Raumgeschwindigkeit von ca. 100 bis 15 000 Liter Gas pro Liter Katalysator und Stunde einführt.

12. Verfahren gemäß den Ansprüchen 7 bis 11, dadurch gekennzeichnet, daß bei Drucken von 0,5 bis 5 bar gearbeitet wird.

13. Verfahren nach Anspruch 1, dadurch gekennzeichnet, daß der Metalloxid-Katalysator ein Metall der Gruppe Magnesium, Calcium, Aluminium enthält.

**Claims**

1. Process for the oxidative dehydrogenation of isobutyric acid to form methacrylic acid using a substantially sulphate-free catalyst based on molybdenum oxide which additionally contains vanadium, phosphorus and another metal, characterised in that the catalyst corresponds to the formula

$$M_aMo_bV_cP_dO_e$$

wherein M represents one of the elements K, Rb, Cs, Be, Mg, Ba, Zn, Ca, Ag, Al, Ti, Pb, Mn, Fe, Co, Ni or Sn and a has a value of from 0.4 to 2.5, b has a value of about 12, c has a value of from 1 to 2 and d has a value of from 0.5 to 3 and e results automatically from the valencies and proportions of the other elements.

2. Process as claimed in claim 1, characterised in that the metal oxide catalyst corresponds to the formula

$$A_{1.5-2}Mo_{12}V_{1.5}P_2O_{45-46}$$

wherein A represents an alkali metal selected from the group comprising potassium, rubidium and caesium.

3. Process as claimed in claims 1 and 2, characterised in that the metal oxide catalyst is used in conjunction with a solid carrier based on silicon dioxide/activated silicon dioxide, preferably based on Kieselguhr/Aerosil®.

4. Process as claimed in claim 3, characterised in that the ratio of silicon dioxide to activated silicon dioxide in the carrier is from 14 : 2 to 9 : 2, preferably 5 : 1.

5. Process as claimed in claims 1 to 4, characterised in that the metal oxide catalyst makes up 5 to 80 % by weight, preferably 20 to 70 % by weight, based on the total weight (carrier plus metal oxide catalyst).

6. Process as claimed in claims 1 to 5, characterised in that the catalyst is calcined at temperatures of between 200 and 400 °C.

7. Process as claimed in claims 1 to 6, characterised in that it is carried out as a gas phase reaction.

8. Process as claimed in claim 7, characterised in that a gas mixture consisting of isobutyric acid, water, oxygen and nitrogen is used.

9. Process as claimed in claims 7 and 8, characterised in that the molar ratio of isobutyric acid to water to oxygen to nitrogen is (1-2.5) : (0-3) : (1-2) : (4-10), preferably 1 : 2 : 1.7 : 20.

10. Process as claimed in claims 1 to 9, characterised in that the reaction is carried out at temperatures of from 300 to 450 °C, preferably at 340 to 370 °C.

11. Process as claimed in claim 8, characterised in that the gas mixture is introduced at a rate by volume of about 100 to 15,000 litres of gas per litre of catalyst per hour.

12. Process as claimed in claims 7 to 11, characterised in that the operation is carried out at pressures of from 0.5 to 5 bar.

13. Process as claimed in claim 1, characterised in that the metal oxide catalyst contains a metal selected from the group comprising magnesium, calcium and aluminium.

**Revendications**

1. Procédé pour la déshydrogénation oxydante de l'acide isobutyrique en acide méthacrylique par utilisation d'un catalyseur exempt de sulfate à base d'oxyde de molybdène contenant en outre du vanadium, du phosphore et un autre métal, caractérisé en ce que le catalyseur répond à la formule

$$M_aMo_bV_cP_dO_e$$

dans laquelle M représente l'un des éléments K, Rb, Cs, Be, Mg, Ba, Zn, Ca, Ag, Al, Ti, Pb, Mn, Fe, Co, Ni ou Sn et a a une valeur de 0,4 à 2,5, b est égal à 12, c a une valeur de 1 à 2 et d'une valeur de 0,5 à 3, la valeur de e étant déterminée par les valences et les proportions des autres éléments.

2. Procédé selon la revendication 1, caractérisé en ce que le catalyseur à base d'oxyde métallique répond à la formule

$$A_{1,5-2}Mo_{12}V_{1,5}P_2O_{45-46}$$

dans laquelle A représente un métal alcalin du groupe du potassium, du rubidium et du caesium.

3. Procédé selon les revendications 1 et 2, caractérisé en ce que le catalyseur à base d'oxydes métalliques est utilisé en combinaison avec un support solide à base de silice/silice activée, de préférence à base de silice/Aerosil (marque déposée).

4. Procédé selon la revendication 3, caractérisé en ce que le rapport silice/silice activée dans le support est de 14 : 2 à 9 : 2, de préférence de 5 : 1.

5. Procédé selon les revendications 1 à 4, caractérisé en ce que le catalyseur à base d'oxydes métalliques représente de 5 à 80 %, de préférence de 20 à 70 % du poids total du support plus catalyseur à base d'oxydes métalliques.

6. Procédé selon les revendications 1 à 5, caractérisé en ce que l'on calcine le catalyseur à des températures de 200 à 400 °C.

7. Procédé selon les revendications 1 à 6, caractérisé en ce qu'il est mis en œuvre sous la forme d'une réaction en phase gazeuse.

8. Procédé selon la revendication 7, caractérisé en ce que l'on opère avec un mélange gazeux d'acide isobutyrique, d'eau, d'oxygène et d'azote.

9. Procédé selon les revendications 7 et 8, caractérisé en ce que les proportions molaires acide isobutyrique/eau/oxygène/azote sont de (1 à 2,5) : (0 à 3) : (1 à 2) : (4 à 10), de préférence de 1 : 2 : 1,7 : 20.

10. Procédé selon les revendications 1 à 9, caractérisé en ce que la réaction est effectuée à des températures de 300 à 450 °C, de préférence de 340 à 370 °C.

11. Procédé selon la revendication 8, caractérisé en ce que l'on introduit le mélange gazeux à une vitesse spatiale horaire d'environ 100 à 15.000 litres de gaz par litre de catalyseur et par heure.

12. Procédé selon les revendications 7 à 11, caractérisé en ce que l'on opère à des pressions de 0,5 à 5 bars.

13. Procédé selon la revendication 1, caractérisé en ce que le catalyseur à base d'oxydes métalliques contient un métal du groupe du magnésium, du calcium, de l'aluminium.